# EUROPEAN PATENT SPECIFICATION

(11) **EP 3 318 204 B1**
(45) Date of publication and mention of the grant of the patent: **12.02.2020**
(21) Application number: 16197716.0
(22) Date of filing: 08.11.2016
(51) Int. Cl.: A61B 17/22, A61B 17/225, G10K 15/04, A61B 17/00

(54) **SHOCKWAVE GENERATOR**
STOSSWELLENGENERATOR
GÉNÉRATEUR D'ONDES DE CHOC

(43) Date of publication of application: 09.05.2018
(73) Proprietor: Enraf Nonius B.V., 3047 AT Rotterdam (NL)
(72) Inventor: STIKVOORT, Raymundus Johannes, 3047 AT Rotterdam (NL); NOMEN, Wessel, 3047 AT Rotterdam (NL)
(74) Representative: De Vries & Metman

(56) References cited:
- EP-A2- 1 426 014
- DE-A1-102004 042 895
- DE-U1-202010 009 899
- US-A- 6 149 656
- US-A1- 2015 063 624

## Description

The present invention relates to an electromagnetic shockwave generator. Such devices are used for therapeutic purposes and typically comprise a handheld piece comprising a central channel and an impactor movable up and down in the channel by electromagnetic interaction. At one end of the channel the impactor impacts an applicator head and generates a mechanical pressure wave to be transferred to a patient's body part. The mechanical energy is absorbed by the patient's tissue and triggers reactions in the tissue that have a positive influence on a wide range of orthopedic and neurological conditions.

DE 10 2004 042895 A1 discloses a shock wave generator for therapeutic treatment with a central bore accommodating an axially movable element for creating shock waves. This element is accelerated by a pneumatic or electromagnetic accelerator.

EP 1 426 014 A2 discloses an endoscopic lithotripsy probe for crushing urinary calculi. The probe comprises a ultrasonic-vibration source and a mechanical shock generation source.

It is an object of the invention to provide a shockwave generator generating shockwaves with reduced loss of kinetic energy, preferably without loss of kinetic energy.

The object of the invention is achieved with a shockwave generator according to claim 1. This way, the same magnets are used to accelerate the impactor into the return direction and to accelerate the impactor into the impacting direction. This reduces loss of kinetic energy.

The coil magnets can for example be annular coils, integral in the wall of the channel. The channel may for example be a cylindrical channel allowing sliding movement of a cylindrical impactor. Optionally, the channel may have a low friction and wear resistant inner surface, e.g. of PTFE, suitable low friction ceramics or fiber reinforced plastic.

In a specific embodiment the shockwave generator may comprise one coil magnet near the applicator head and one coil magnet near the end section, so the impactor is accelerated in the desired direction over its entire path.

The impactor can for example be a permanent magnet, e.g., a ring magnet. A ring magnet encounters less air resistance when it is moved through the channel. Suitable materials for such a magnet include alloys of iron and neodymium, in particular of the N50 grade, although other suitable alloys can also be used.

To bounce back the impactor when it approaches the end section of the channel, the end section may for example comprise a bouncer, such as a permanent magnet, e.g., a ring magnet or disc magnet, with a polarity opposite to the polarity of the impactor. Such a permanent magnetic bouncer has the advantage that no kinetic energy is lost during bouncing. All energy can be transferred to the applicator head. The magnetic field of the bouncer magnet has approximately the same strength as the magnetic field of the impactor magnet, or it may be stronger.

By activating the front coil magnet the impactor will be moved by the bouncer to a position against the applicator head. When the shockwave is turned on for use, this front coil magnet is activated with a polarity opposite to the polarity of the impactor. The generated magnetic field thrusts the impactor towards the rear coil magnet, which is subsequently or simultaneously activated to have a polarity which is opposite to the polarity of the front coil magnet, so it attracts the impactor and helps to accelerate it.

When the motion of the impactor is reversed by the bouncer magnet at the end section of the channel, the polarity of the two coil magnets is reversed: the polarity of the rear coil magnet being opposite to the polarity of the impactor in order to repel the impactor, while the polarity of the front coil magnet is such that it attracts the impactor. The impactor is bounced back by the bouncer magnet and is again accelerated by the magnetic fields of the two coil magnets.

The impactor impacts a shock transducer comprising a contact face at the outside of the shock wave generator for transducing the shockwave to a patients' body part. The shock transducer may for example be moveable relative to the applicator body under the action of the impactor. The shock transducer may for example be made of a plastic material, such as POM or reinforced plastic. Such materials were found to have good resiliency to transduce the shocks to a patient.

The shockwave generator may for example comprise a sliding guide limiting movement of the shock transducer relative to the applicator body. In a particular embodiment the sliding guide may comprise a flange of the shock transducer slidingly received in a recess in an inner surface of the applicator body. This way, the shock transducer is moveable, e.g., between a retracted position and a projecting position relative to the applicator body. The length of the recess in moving direction of the impactor defines the amplitude of the shock transducer's movement.

The contact face of the shock transducer may for example be convex, e.g., being flush with the applicator body when the shock transducer is in the retracted position.

After the impactor impacts the shock transducer, the coil magnets are again activated to move and accelerate the impactor for a next turn. The shockwave generator can be operated with any desired impacting frequency, for instance 20 times per second.

The invention will be further explained with reference to the drawings, showing an exemplary embodiment.
- Figure 1:: shows an exemplary embodiment of a shockwave generator of the present invention;
- Figures 2-7:: show consecutive steps of using the shockwave generator of Figure 1.

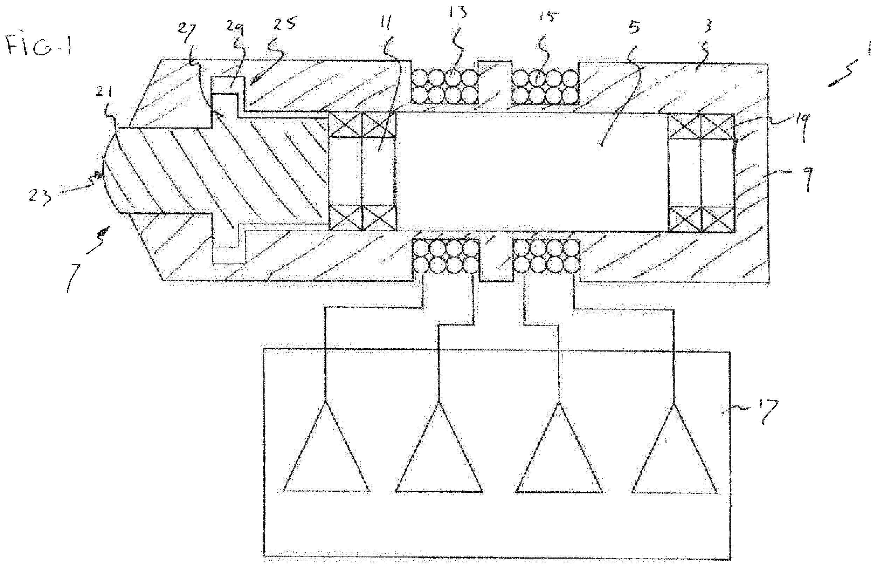

Figure 1 shows a shockwave generator 1 comprising a cylindrical body 3 of a plastic or ceramic material with a cylindrical channel 5 extending between an applicator head 7 and a closed end section 9. In an alternative embodiment, the end section may be an open end section and/or the body may be non-cylindrical. Also other materials may be used. An electromagnetically driveable impactor 11, formed as a permanent ring magnet or disc magnet, is slideable in the channel 5 between a position at the applicator head 7 and a position at the end section 9.

The channel 5 has channel walls embedding two coil magnets: a front coil magnet 13 and a rear coil magnet 15, both coaxially surrounding the channel 5. The front coil magnet 13 is located near the applicator head 7 and is aligned with the impactor 11 when the impactor 11 is in its start position against the applicator head 7. The rear coil magnet 15 is closer near the end section 9 of the channel.

The shockwave generator 1 further comprises a control unit 17 programmed and configured to reverse the polarity of the front and rear coil magnets 13,15 so as to optimize acceleration of the impactor 11.

The end section 9 of the channel 5 is provided with a bouncer 19 formed by a ring magnet or disc magnet of approximately equal size as the ring magnet or disc magnet forming the impactor 11, but having an opposite polarity. In an alternative embodiment, the bouncer 19 may be larger or smaller than the impactor 11.

The applicator head 7 comprises a shock transducer 21 which is, e.g., wholly or partly made of a plastic material and which is moveable relative to the applicator body 3 under the impacting action of the impactor 11. The shock transducer 21 comprises a convex contact face 23 at the outside of the shockwave generator 1. A sliding guide 25 within the channel 5 limits movement of the shock transducer 21 relative to the applicator body 3. This sliding guide 25 comprises an annular flange 27 of the shock transducer 21 slidingly received in an annular recess 29 in an inner surface of the channel 5. The shock transducer 21 is moveable between a retracted position and a projecting position (as in Figure 1) relative to the applicator body 3.

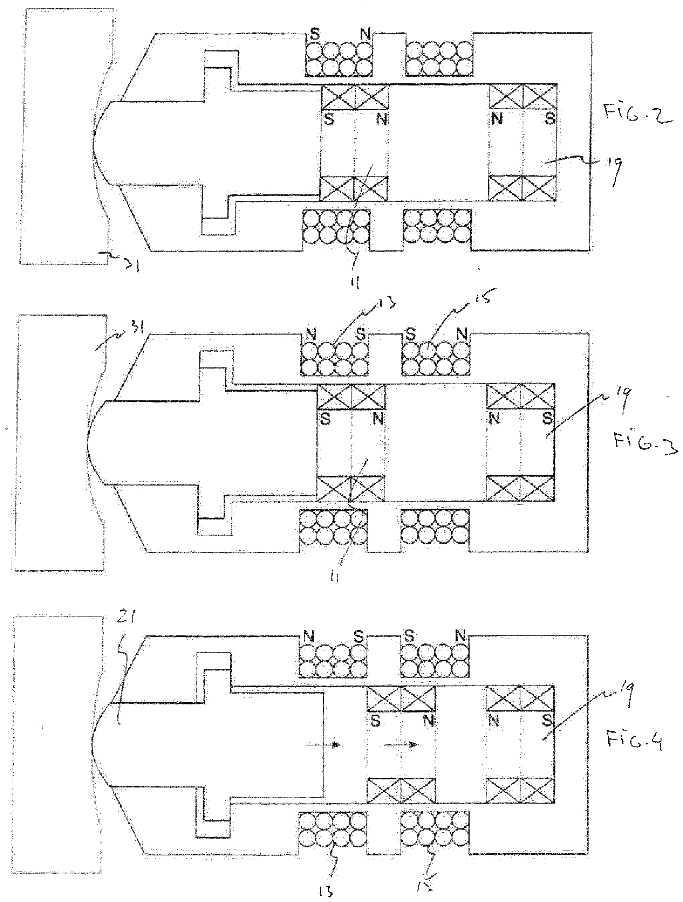

Figure 2 shows the situation when the shockwave generator 1 is positioned on a patient's body part 31 and the front coil magnet 13 is magnetized with the same polarity as the impactor magnet 11, thus pulling the impactor against the shock transducer 21. The rear coil magnet 15 is still deactivated.

In Figure 3, the polarity of the front coil magnet 13 is reversed and the rear coil magnet 15 is activated and oppositely polarized. The front coil magnet 13 now pushes the impactor 11 away from the applicator head 7 towards the bouncer magnet 19 and the rear coil magnet 15 pulls the impactor 11 into the same direction until the rear end of the impactor 11 is fully encircled by the front side of the rear coil magnet 15. The shock transducer 21 can now be moved inwardly by the pressure exerted by the patient's body part 31 (Figure 4).

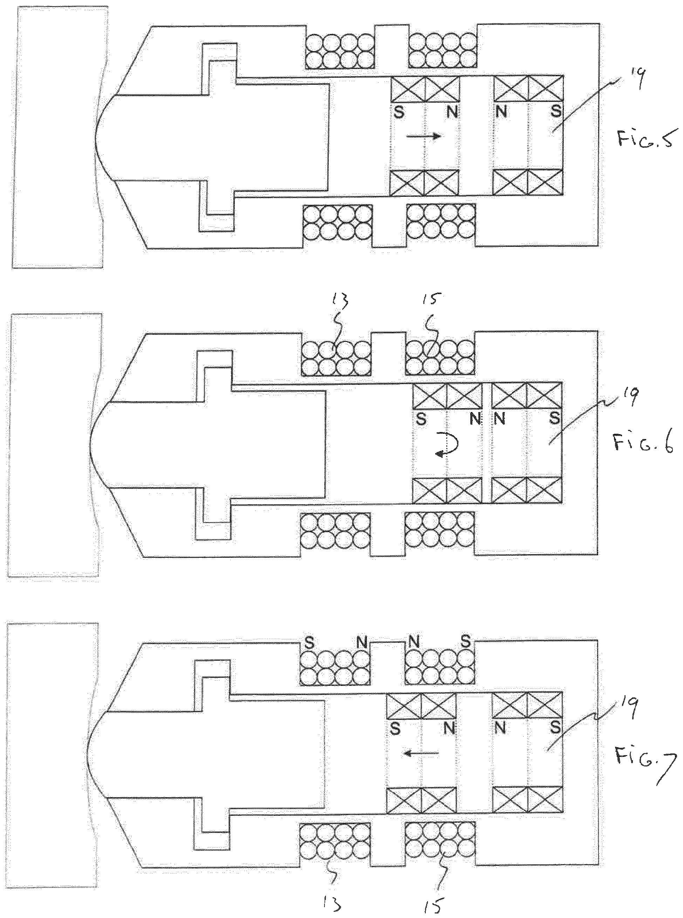

In a next step, the coil magnets 13, 15 are deactivated (Figure 5) to allow the impactor magnet 11 to slide with a maximum kinetic energy towards the bouncer magnet 19.

Figure 6 shows the moment that the impactor 11 is reversed when it approaches the bouncer magnet 19 at the end section of the channel 5. The impactor magnet 11 returns without contacting the bouncer magnet 19. Subsequently (Figure 7), the two coil magnets 13, 15 are again activated with a polarity which is opposite to the polarity in the stage of Figures 3 and 4. : the polarity of the rear coil magnet 15 is now opposite to the polarity of the impactor 11 in order to repel the impactor 11, while the polarity of the front magnet 13 is the same as the impactor's polarity such that it attracts the impactor magnet 11. The impactor magnet 11 is bounced back by the bouncer magnet 19 and is again accelerated by the magnetic fields of the two coil magnets 13, 15. The impactor 11 hits the shock transducer 21, which resiliently bounces in the sliding guide 25 and transduces the generated shockwave to the patient's skin 31. The cycle of steps can then be restarted.

## Claims

1. Shockwave generator (1) comprising a body (3) with a channel (5) extending between an applicator head (7) and an end section (9), an electromagnetically driveable impactor (11) slideable in the channel,
wherein the applicator head of the body (3) comprises a shock transducer (21) moveable relative to the applicator body under the action of the impactor, the shock transducer comprising a contact face (23) at the outside of the shock wave generator,
**characterized in that** the shockwave generator comprises at least two serially arranged coil magnets (13, 15) with a reversible polarity for thrusting the impactor,
wherein the shockwave generator comprises a control unit (17) programmed to reverse the polarity of the coil magnets.

2. Shockwave generator according to any preceding claims, wherein the coil magnets (13, 15) are annular coils integral in the wall of the channel.

3. Shockwave generator according to any preceding claim, comprising one coil magnet (13) near the applicator head and one coil magnet near (15) the end section (9).

4. Shockwave generator according to any preceding claim, wherein the impactor (11) is a permanent magnet, e.g., a ring magnet or a disc magnet.

5. Shockwave generator according to claim 4, wherein the end section (9) comprises a permanent magnet, e.g., a ring magnet or disc magnet, with a polarity opposite to the polarity of the impactor.

6. Shockwave generator according to claim 1, wherein the shock transducer (21) is made of a plastic material.

7. Shockwave generator according to claim 5 or 6, comprising a sliding guide (25) limiting movement of the shock transducer (21) relative to the applicator body (3).

8. Shockwave generator according to claim 7, wherein the sliding guide (25) comprises a flange of the shock transducer (21) slidingly received in a recess in an inner surface of the applicator body.

9. Shockwave generator according to claim 8, wherein the shock transducer (21) is moveable between a retracted position and a projecting position relative to the applicator body (3).

10. Shockwave generator according to any one of claims 5-9, wherein the contact face (23) of the shock transducer is convex.

## Patentansprüche

1. Stoßwellengenerator (1), aufweisend einen Körper (3) mit einem Kanal (5), der zwischen einem Applikatorkopf (7) und einem Endabschnitt (9) verläuft, einen elektromagnetisch ansteuerbaren Stoßkörper (11), der in dem Kanal verschiebbar ist,
wobei der Applikatorkopf des Körpers (3) einen Stoßwandler (21) aufweist, der unter Einwirkung des Stoßkörpers relativ zum Applikatorkörper bewegbar ist, wobei der Stoßwandler eine Kontaktfläche (23) an der Außenseite des Stoßwellengenerators aufweist,
**dadurch gekennzeichnet, dass** der Stoßwellengenerator mindestens zwei in Serie angeordnete Spulenmagnete (13, 15) mit umkehrbarer Polarität zum Stoßen des Stoßkörpers aufweist,
wobei der Stoßwellengenerator eine Steuereinheit (17) aufweist, die zum Umkehren der Polarität der Spulenmagnete programmiert ist.

2. Stoßwellengenerator nach einem der vorstehenden Ansprüche, wobei die Spulenmagnete (13, 15) ringförmige Spulen sind, die in einem Stück mit der Wand des Kanals gebildet sind.

3. Stoßwellengenerator nach einem der vorstehenden Ansprüche, der einen Spulenmagnet (13) nahe dem Applikatorkopf und einen Spulenmagnet (15) nahe dem Endabschnitt (9) aufweist.

4. Stoßwellengenerator nach einem der vorstehenden Ansprüche, wobei der Stoßkörper (11) ein Permanentmagnet, z. B. ein Ringmagnet oder ein Scheibenmagnet ist.

5. Stoßwellengenerator nach Anspruch 4, wobei der Endabschnitt (9) einen Permanentmagnet, z. B. einen Ringmagnet oder einen Scheibenmagnet, aufweist, dessen Polarität der Polarität des Stoßkörpers entgegengesetzt ist.

6. Stoßwellengenerator nach Anspruch 1, wobei der Stoßwandler (21) aus einem Plastikmaterial gefertigt ist.

7. Stoßwellengenerator nach Anspruch 5 oder 6, der eine Gleitführung (25) aufweist, die die Bewegung des Stoßwandlers (21) relativ zum Applikatorkörper (3) begrenzt.

8. Stoßwellengenerator nach Anspruch 7, wobei die Gleitführung (25) einen verschiebbar in einer Ausnehmung in einer Innenfläche des Applikatorkörpers aufgenommenen Flansch des Stoßwandlers (21) aufweist.

9. Stoßwellengenerator nach Anspruch 8, wobei der Stoßwandler (21) zwischen einer eingezogenen Position und einer hervorstehenden Position in Bezug auf den Applikatorkörper (3) bewegbar ist.

10. Stoßwellengenerator nach einem der Ansprüche 5 - 9, wobei die Kontaktfläche (23) des Stoßwandlers konvex ist.

## Revendications

1. Générateur d'ondes de choc (1) comprenant un corps (3) avec un canal (5) s'étendant entre une tête d'applicateur (7) et une section d'extrémité (9), un impacteur pilotable électromagnétiquement (11) pouvant coulisser dans le canal,
dans lequel la tête d'applicateur du corps (3) comprend un transducteur de choc (21) mobile par rapport au corps d'applicateur sous l'action de l'impacteur, le transducteur de choc comprenant une face de contact (23) à l'extérieur du générateur d'ondes de choc,
**caractérisé en ce que** le générateur d'ondes de choc comprend au moins deux aimants de bobine agencés en série (13, 15) de polarité inversable pour pousser l'impacteur,
dans lequel le générateur d'ondes de choc comprend une unité de commande (17) programmée pour inverser la polarité des aimants de bobine.

2. Générateur d'ondes de choc selon une quelconque revendication précédente, dans lequel les aimants de bobine (13, 15) sont des bobines annulaires d'un seul tenant dans la paroi du canal.

3. Générateur d'ondes de choc selon une quelconque revendication précédente, comprenant un aimant de bobine (13) près de la tête d'applicateur et un aimant de bobine (15) près de la section d'extrémité (9).

4. Générateur d'ondes de choc selon une quelconque revendication précédente, dans lequel l'impacteur (11) est un aimant permanent, par exemple, un aimant en forme d'anneau ou un aimant en forme de disque.

5. Générateur d'ondes de choc selon la revendication 4, dans lequel la section d'extrémité (9) comprend un aimant permanent, par exemple, un aimant en forme d'anneau ou un aimant en forme de disque, de polarité opposée à la polarité de l'impacteur.

6. Générateur d'ondes de choc selon la revendication 1, dans lequel le transducteur de choc (21) est réalisé en un matériau plastique.

7. Générateur d'ondes de choc selon la revendication 5 ou 6, comprenant un guide coulissant (25) limitant un déplacement du transducteur de choc (21) par rapport au corps d'applicateur (3).

8. Générateur d'ondes de choc selon la revendication 7, dans lequel le guide coulissant (25) comprend une bride du transducteur de choc (21) reçue en coulissement dans un évidement dans une surface intérieure du corps d'applicateur.

9. Générateur d'ondes de choc selon la revendication 8, dans lequel le transducteur de choc (21) est mobile entre une position rétractée et une position saillante par rapport au corps d'applicateur (3).

10. Générateur d'ondes de choc selon l'une quelconque des revendications 5 à 9, dans lequel la face de contact (23) du transducteur de choc est convexe.
